# EUROPEAN PATENT SPECIFICATION

(11) **EP 0 527 975 B1**
(45) Date of publication and mention of the grant of the patent: **02.10.1996**
(21) Application number: 92903780.2
(22) Date of filing: 03.02.1992
(51) Int. Cl.: A61K 31/485, C07D 498/00, C07D 489/08

(54) **Codeinone derivatives and their pharmaceutical use**
Codeinonderivate und ihre pharmazeutische Verwendung
Dérivés de la codéinone et leur utilisation pharmaceutique

(30) Priority: 04.02.1991 HU 36991
(43) Date of publication of application: 24.02.1993
(73) Proprietor: ALKALOIDA CHEMICAL COMPANY LTD., H-4440 Tiszavasvari (HU)
(72) Inventor: BORSODI, Anna, H-6791 Szeged (HU); FÜRST, Zsuzsa, H-1015 Budapest (HU); HOSZTAFI, Sándor, H-4275 Monostorpályi (HU); SCHÄFFERNE VARGA, Eva, H-6791 Szeged (HU); BUZAS, Beáta, H-3300 Eger (HU); FRIEDMANN, Tamás, H-1121 (HU); BENYHE, Sándor, H-6723 Szeged (HU); SZÜCS, Mária, H-6721 Szeged (HU)
(74) Representative: Beetz & Partner Patentanwälte
(86) International application number: PCT/HU92/00006
(87) International publication number: WO 92/13534

(56) References cited:
- EP-A- 0 077 521
- EP-A- 0 242 417
- US-A- 4 760 069
- US-A- 4 889 860

## Description

The present invention relates to novel codeinone derivatives and to the use of codeinone derivatives for preparing pharmaceutical compositions for analgetic treatment without respiratory depression. These products are capable of selectively blocking the opiate-binding sites of the brain which are responsible for respiratory depression.

It is known that the morphine-like analgesics (opiates) act as respiratory depressants. Based on acute toxicity investigations the lethality risk of such compounds is attributed to this activity [Prog. Neurobiol.33(1984)1-16; 22(1984) 345].

The interpretation of relations between respiratory depressant activity and opiate receptors is rather difficult. According to the present concepts there are several receptors which might participate in mediating this effect. However, the relative contribution of various receptor populations mediating this effect is not clear enough. Since evaluating the respiratory parameters is complicated, and the selectivity of the methods is questionable and because of the big differences amongst the species the difficulties are further increased. The difficulties and problems inherent in these methods partially explain the different receptor assignments in opioid-induced respiratory depression which have appeared in the literature.

Several biochemical and pharmacological data prove the heterogeneity of the opioid receptors both in the neurological system and in the peripheries [Trends. Neuro. Sci.7.(1984) 31; Pharmac.Rev.39, 197-249 (1987).]

The main receptors - marked µ, δ , κ and σ receptors - show different dispersity in different tissues, have different ligand specifities and mediate different physiological processes. Recently it was clarified also that these main receptor types can be divided into sub-types. In the case of the µ type receptors it is possible to distinguish between µ₁ and µ₂ sub-types. It is suggested that the µ₁ receptors mediate the analgesic effects of opiates while the µ₂ receptors mediate opioid induced respiratory depression.

The identification of these two sub-types of opioid receptors may be brought about by way of their binding capacity to opioid receptors of so-called high and low affinity. Ligands for µ₁ receptors are bound to the high affinity (³H)naloxone binding sites. (Their affinity constants are below nanomole concentrations.) The µ₂ receptors (low affinity binding sites) mediate, among others, opioid induced respiratory depression. (Their affinity constants are in the range of nanomole concentrations or above.)

Ligands are known which are capable to inhibit selectively the µ₁ receptors. Examples for this group are naloxonazine, oximorphazone, several other C-6 morphinane derivatives and interestingly also some peptides (the chloromethylketones of enkephalins).

As far as we are aware no compounds capable to block the µ₂ receptors were known hitherto. It is a new observation that codeinone, oxycodone and dihydrocodeinone derivatives substituted in C-6 position are bound in a partially irreversible manner to µ₂ receptors.

It has to be mentioned, however, that the existence of µ₁ and µ₂ receptors has not yet found general acceptance. There were several authors who could not repeat the experiment which serves as main proof, namely that the respiratory depression caused by morphine is not inhibited by the selective µ₁ antagonist naloxonazine.

A further difficulty may be caused also by the analysis of the complex action of opiates on respiration. There are opiates known which are depressing ( µ-agonists: morphine, etc.), others which are stimulating (receptor agonists: cyclazocine), and others which have dualistic (deprimating and stimulating) activity (partial antagonists: nalorphine).

The significance of an identification of the µ₂ receptors would be in theory and in practice that knowing the same there would be a possibility to synthesize such compounds which would not have respiratory depressing action while having considerable analgesic properties.

It is also known that there are certain dihydromorphinone and dihydrocodeinone derivatives substituted in C-6 position with hydrazone, phenylhydrazone, dinitrophenylhydrazone, semicarbazone and thiosemicarbazone groups as well as their 14-OH-derivatives possessing increased analgesic activity as compared with the analogues which are 6-unsubstituted (HU-A-56-85, HU 199 901).

The C-6 carbonyl group of the morphinane ketones is easily substituted, and earlier publications suggest that changes of this type in general do not influence the opioid character thereof.

It has been stated when investigating 6-substituted hydrazone derivatives of 14-hydroxydihydromorphinone [J.Med.Chem. 23 (1980) 674-677, Pharm.Res. 3 (1986) 56-60, Life Sci., 40(1987) 1579-1588] that both the µ and the δ high affinity binding sites are irreversibly blocked by these derivatives [J.Pharm. Exp. Ther. (1980) 214 and 455-462]. It has been shown by pharmacological tests that irreversible blocking of these receptors is manifested in the case of agonists in a long-lasting analgesic effect, while in the case of antagonists the analgesic activity of morphine is blocked for a long period of time while, however, the protection against lethal respiratory depression does not take place [Life Sci. 31 (1983) 1389-1392, Science 208 (1989) 514-516]. It has thus been stated that the opiate analgesia is mediated by the common receptors named µ₁.

Thus it would be a most desirable task for pharmaceutical planning to separate the analgetic effect from other opiate effects.

In accordance with the above it is the underlying problem of the present invention to provide codeinone derivatives and the pharmaceutical use of codeinone derivatives for preparing pharmaceutical compositions for analgetic treatment without respiratory depression through selective blocking of the opioid binding sites of the brain responsible for respiratory depression.

The above problem is solved according to the independent claims. The dependent claims relate to preferred embodiments.

The codeinone derivatives of the present invention have the general formula Ia, 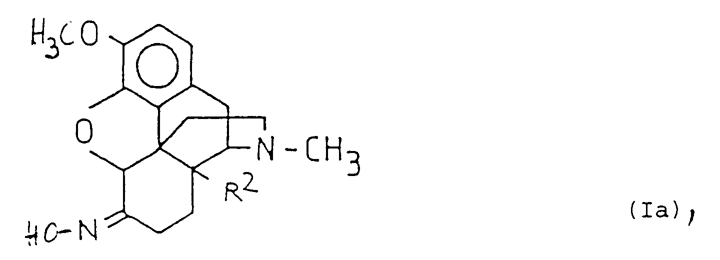 wherein
R² is a hydrogen atom or hydroxy,
including their salts.

The invention is further directed to pharmaceutical compositions comprising a codeinone derivative of formula Ia.

In accordance with a preferred embodiment, the codeinone derivatives of formula Ia are present in the form of their cis and/or trans isomers.

According to another preferred embodiment, the pharmaceutical compositions exhibiting analgesic activity
comprise
(A) a codeinone derivative of the general formula Ia or a salt thereof,
   and
(B) morphine of the formula IV 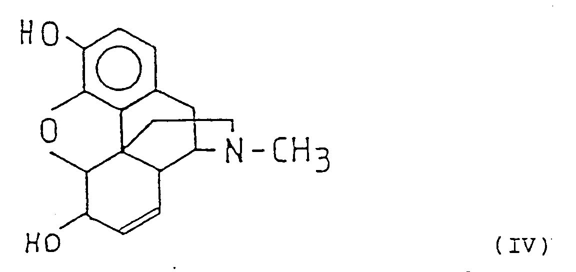 or
   another opiate of agonist type or an opioid compound,
the codeinone derivative of component (A) being present in an amount of 2 to 3 parts by mass per 1 part by mass of morphine or a biologically equipotent amount of non-morphine component (B).

The invention further pertains to a process for the preparation of the codeinone derivatives of the general formula Ia which is
characterized in that,
in a manner known as such, a ketone of the general formula II, 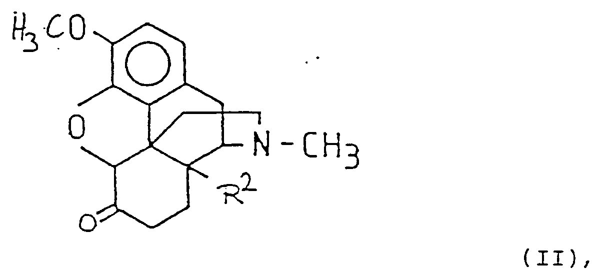 wherein
R² is a hydrogen atom or hydroxy,
is allowed to react with a hydrazine derivative of the general formula IIIa,

H₂N-R^{3'} (IIIa),

wherein
R^{3'} is hydroxy or
a group capable of being transformed into hydroxy.

The present invention is further directed to the use of codeinone derivatives of the general formula I, 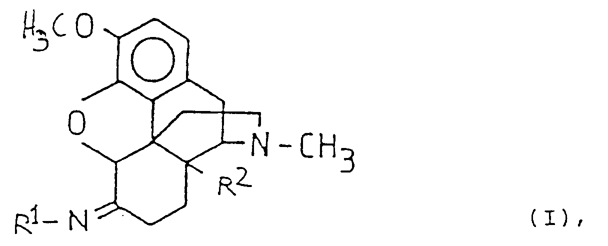 wherein
R¹ is amino, hydroxy, -NH-phenyl or -NH-CO-NH₂,
   and
R² is a hydrogen atom or hydroxy,
including their salts,
for preparing pharmaceutical compositions for analgesic treatment without respiratory depression.

When starting the work according to the present invention, the effect of different C-6 substituted (hydrazone, phenylhydrazone, dinitrophenylhydrazone, oxime, semicarbazone) derivatives of dihydromorphinone and dihydrocodeinone on brain membranes of rats have been studied. The C-6 substitution did not change their affinities to the (³H)naloxone-binding sites in the case of substituents of small size, while the affinity decreased in the case of substituents having a greater volume.

It was stated that all tested C-6 substituted derivatives of oxymorphone and dihydromorphinone are blocking irreversibly, in a dose-dependent manner the specific binding of (³H)naloxone at isotope concentrations of 1nM, and this concerns the µ₁ receptor.

In accordance with the present invention it has been found surprisingly that oxycodone and dihydrocodeinone derivatives of the (general formula I are irreversible inhibitors in a dose-dependent manner of the specific binding of (³H)naloxone at isotope concentrations of 1nM and that they have a lower affinity to the (³H)naloxone binding sites as compared with the corresponding morphine derivatives.

However, it was ascertained that at isotope concentrations of 10 nM preincubation with these dihydrocodeinone derivatives blocks the specific binding of (³H)naloxone irreversibly and completely, as well seen from the saturation isotherms of (³H)naloxone (preincubation of the membranes with these dihydrocodeinone derivatives followed by washing). It is essential that this involves the binding sites of low affinity (µ₂) which are responsible for respiratory depression.

In connection with the present invention, it was investigated in pharmacological tests based on the above whether the derivatives of the general formula I would be exempt from respiratory depression actions which are presumably mediated by the µ₂ receptors. Starting from the fact that the toxicity of morphine is attributed to respiratory paralysis toxicity tests with the above compounds and morphine have been conducted on rats to get indirect information on the nature of the respiratory depressant activity. From the classical opiate spectrum the analgetic effect and the sedative action have been investigated.

It was verified that the codeinone derivatives of the general formula I significantly inhibit the lethal respiratory depression caused by morphine (e.g. on rats), leading to LD₅₀ values measured on morphine controls increased in the average by a factor of 2.

On the basis of the tables shown in the experimental section it can also be stated that a part of the compounds stimulated in small dose ranges the frequency of respiration and some even the amplitude, administered i.v. and s.c. For example the oxycodone-semicarbazone and -oxime when used in small doses considerably increase the respiration number, and in some tests they reduced the depressant effect of morphine. In all experiments respiration was reversed with naloxone to prove that the depression was mediated by an opiate receptor. When administering together the morphine effect was slightly inhibited at doses of 100 µg/kg. Oxycodone hydrazone increases both the volume and the frequency of respiration at 50 µg/kg doses. When administered after morphine a partial reversal takes place. While somewhat depressing respiration oxycodone-phenylhydrazone partially antagonizes the effect of morphine. It has to be mentioned that amongst the "parent compounds" dihydrocodeinone and oxycodone referred to cause respiratory depression at doses of 50-2500 µg/kg and 10-2500 µg/kg s.c., respectively. Both compounds unambiguously potentiate the respiratory depression effect of morphine.

The above experiments suggest that the codeinone derivatives of general formula I according to our invention block selectively the opioid binding sites of the brain which are mediating respiratory depression, and thus they can be used both in human and in veterinary preparations in all cases where such blocking is necessary.

EP-A-077 521 discloses opiate agonists and antagonists and their use as opiate receptor blockers. These compounds are dihydromorphinone derivatives.

EP-A-242 417 relates to morphinone-based hydrazone derivatives possessing analgetic or morphine-antagonistic activity.

It could not be expected from this prior art that the compounds of formula I would be even able not only to exhibit an analgetic activity, but, at the same time, to inhibit respiratory depression caused by the opioid analgesics when administered together.

Thus the codeinone derivatives of general formula I can be used as active ingredients in analgesic preparations without the appearance of lethal respiratory depression after longer treatment or higher doses.

The derivatives of general formula I can also be used preferably in combinations according to the present invention. Thus it is specifically important that when administered together with morphine the respiratory depression can be decreased, and the lethal effect can be avoided. Thus it is possible to administer morphine in prolonged and higher doses in cases where the patient needs it without the occurrence of lethal respiratory paralysis.

In preparations where morphine and the codeinones of the general formula Ia are applied together the mass proportion of morphine : codeinone derivative amounts to 1 : 2-3.

Instead of morphine biologically equipotent amounts of other opiates of the agonist type or opioid compounds can be used in these combination preparations. Such compounds are e.g. morphinans, benzomorphans, opioid peptides or other pentacyclic compounds with morphine activity.

In addition to the above active ingredients the compositions according to the present invention contain inert, pharmaceutically acceptable additives.

For selectively blocking the opioid binding sites of the brain which are responsible for the depression of respiration, a composition containing a codeinone derivative of general formula I or a salt thereof is administered to the patient in need of such treatment, preferably in a 3x2,5 - 5 mg daily dose.

It is preferable to use the product in the form of i.m., i.v., or epidural injections; however, it is possible to use other administration forms as well.

For the preparation of biologically active compositions containing codeinone derivatives of general formulae I or Ia the codeinone derivatives are admixed, alone or together with morphine or some other opiate or opioid compound of the agonist type with additives or auxiliary compounds usually applied in the pharmaceutical production for human or veterinary purposes so as to formulate compositions capable to block the opioid binding sites responsible for respiratory depression, preferably analgesics.

According to the present invention the codeinone derivatives of the general formulae I and Ia can be present in the form of their salt formed with mineral or organic acids, preferably phosphoric acid or hydrochloric acid.

According to this invention it is possible to use the compounds in the form of their cis and/or trans isomers.

As compared with the HU-A-199.901 the present invention represents a distinct, limited selection of 6-substituted derivatives.

It is not possible to use all the molecules of the big group of morphinan derivatives. Thus, the selective binding utilized according to the present invention represents a property, the recognition of which constitutes the essential basis of the invention which has been accomplished through the selection of outstandingly useful compounds out of the bigger range of others.

Details of the present invention are illustrated in the Examples.

### I. Examples for compositions

Compositions containing the following active ingredients are prepared with usual additive materials and methods:

**Table**

| Compound | | size (mg/unit) (tablet or capsule) | dose of active ingredient (mg of base) | | |
|---|---|---|---|---|---|
| | | | i.m. or s.c. inj. | i.v. inj. | epidur. inj. |
| I.1. | Oxycodone-oxime phosphate | 5.0 | 2.0 | 1.0 | 0.5 |
| I.2. | Oxycodone-oxime .HCl + morphine.HCl | 2.0 | 0.5 | 0.1 | 0.1 |
| | | 5.0 | 5.0 | 5.0 | 1.0 |
| I.3. | Oxycodone-semicarbazone-bitartarate | 2.5 | 1.0 | 0.5 | 0.1 |
| I.4. | Oxycodone-phenylhydrazone.HCl | 2.5 | 1.0 | 0.5 | 0.1 |
| I.5. | Oxycodone-hydrazone.HBr | 2.5 | 1.0 | 0.5 | 0.1 |
| I.6. | Oxycodone-semicarbazone.HCl + morphine.HCl | 2.5 | 1.0 | 0.5 | 0.1 |
| | | 5.0 | 5.0 | 5.0 | 1.0 |
| I.7. | Oxycodone-phenylhydrazone.HCl + morphine.HCl | 2.5 | 1.0 | 0.6 | 0.1 |
| | | 5.0 | 5.0 | 5.0 | 1.0 |
| I.8. | Oxycodone-hydrazone.HCl + morphine.HCl | 2.5 | 1.0 | 0.5 | 0.1 |
| | | 5.0 | 5.0 | 5.0 | 1.0 |
| I.9. | Dihydrocodeinone-oxime phosphate | 10.0 | 5.0 | 2.0 | 1.0 |
| I.10. | Dihydrocodeinone-oxime.HCl + morphine.HCl | 5 | 2.5 | 1.0 | 0.1 |
| | | 5.0 | 5.0 | 5.0 | 1.0 |

The dose of the active ingredient is calculated as base, the size is mg/formulated unit (tablet, ampoule).

### Example I.11.

Tablets of the following composition are prepared with usual methods:

| | |
|---|---|
| Salt of the active ingredient | 5.0 mg (calculated as base) |
| lactose | 60.0 mg |
| starch | 30.0 mg |
| magnesium stearate | 1,0 mg |
| talc | 3,0 mg. |

### II. Binding tests

### Methods:

Membrane preparation: A crude membrane fraction from rat brain (PVG/C strain) was prepared [Mol.Pharm.11 (1975) 340-351], and the protein content was defined [Anal. Biochem.72(1976) 248-254).

### Description of the binding assay:

The membrane suspension (200-400 µg of protein) was incubated with the specific radioligand for 1 hour on ice. Incubations were terminated by rapid filtration under vacuum followed by washing with ice-cold tris-HCl buffer (50 mM, pH 7.4). Radioactivity was measured in a toluene-based scintillation mixture on an LKB Minibeta Liquid Scintillation Spectrophotometer. Nonspecific binding was defined in the presence of 10 µM unlabeled naloxone. All assays were performed in triplicate and repeated several times. Kᵢ values were determined from equilibrium experiments and calculated with the Tshang-Prusov equation. The data were evaluated using the Ligand program [Anal.Biol. Chem. 107 (1980) 220 - 239] (Data shown on Table III/1).

### Determination of wash-resistant binding:

After preincubation a thorough washing was performed [ Life Sci.32 (1983) 2777- 2784]. Control values are represented by the specific binding of (³H)naloxone to membranes preincubated with a buffer and treated in the same way. Heterologous displacement experiments were used to evaluate the affinity of the investigated compounds for (³H)naloxone binding sites. Results are shown in Table II/2.

### II.1. Binding tests

**Table II/1**

| Affinity constants (KᵢnM) of different opioid ligands in competition assays | | | | | |
|---|---|---|---|---|---|
| | hydrazone | phenylhydrazone | semi carbazone | oxime | salt |
| oxymorphone | 2 | 20 | 4 | 2 | sulfate |
| dihydromorphinone | 6 | 5 | 3 | 2 | sulfate |
| oxycodone | 800 | 1150 | 588 | 32 | HCl |
| dihydrocodeinone | - | 323 | - | 52 | HCl |
| membranes were incubated with (³H)naloxone (1nM) and with increasing concentrations of the ligands. | | | | | |

### Evaluation:

The oxime derivatives exhibit the highest affinity for (³H)naloxone binding sites. The codeinone and dihydrocodeinone derivatives have substantially higher Kᵢ values.

### II.2. Assay on wash-resistant binding

**Table II/2**

| Affinity constants of oxycodone and dihydrocodeinone derivatives | | |
|---|---|---|
| Example | compound.HCl | Kᵢ(nM) |
| - | oxycodone | 127 |
| IV.2. | oxycodone-oxime | 32 |
| IV.5. | oxycodone-semicarbazone | 588 |
| IV.6. | oxycodone-phenylhydrazone | 1150 |
| - | dihydrocodeinone | 476 |
| IV.1. | dihydrocodeinone-oxime | 53 |
| | dihydrocodeinone-phenylhydrazone | 323 |
| The membranes were preincubated with (³H)naloxone (1 nM) and with increasing concentrations of the tested compounds, and the specific binding of the remaining (³H)naloxone was measured after several washings. The data given represent the average of 2-4 data observed. | | |

The codeinone derivatives were measured at 10 µM preincubation concentrations. Evaluation: Diagram V. shows that the compounds of the general formula I are weak, irreversible inhibitors of specific binding of (³H)naloxone at a concentration of 1 nM, especially on high activity receptors. At higher concentrations the situation is reversed, and they inhibit mainly the low affinity receptors.

As it is seen in Diagram VI. the remaining (³H)naloxone specific binding is significantly decreased at an isotope concentration of 10 nM as a result of preincubation with dihydrocodeinone derivatives, while this decrease is rather small if preincubation takes place with dihydromorphinone derivatives.

It was already shown previously [Life Sci.40 (1980) 1579-1588, J.Pharm.Exp. Ther.214 (1980) 455-462] that preincubation with hydrazone derivatives of oxymorphone and naloxone irreversibly inhibits the high affinity (³H)naloxone component, the low affinity component remaining unchanged. As may be seen from Table II/1., the oxycodone and dihydrooxycodone derivatives show a lower affinity to the (³H)naloxone binding sites as compared with the correspondent morphine derivatives, if the isotope concentration is low.

These ligands however strongly inhibit the (³H)naloxone specific binding at higher concentrations ( 10 nM). This is why the effect of preincubation with these derivatives has been studied on saturation isotherms of (³H)naloxone.

Figure VII. shows the Scatchard transformations of the saturation binding isotherms of (³H)naloxone specific binding after pretreatment with dihydrocodeinone derivatives and with buffer only as a control. From the Scatchard analysis the result is clearly seen, that after preincubation and intensive washings the low affinity receptor-effect disappears almost completely.

### III. Pharmacological tests

It is characteristic for the compounds that their greater part shows a substantial and some a light agonist ( analgetic or sedative) effect (Tables No. I, II, III).

### III.1. Analgetic effects

Comparison on the basis of data obtained in 4 tests on rats and mice (ED₅₀,mg/kg):

**Table III/1**

| compound | hot plate (rat) | tail flick (rat) | writhing test (mice) | algolyt. test (rat) 100% activity |
|---|---|---|---|---|
| oxycodone-oxime phosphate | 1.8 | 1.0 | 0.11 | 10.0* |
| oxycodone-semicarbazone bitartarate | 0.58 | 0.35 | 0.15 | 5.0* |
| oxycodone-phenylhydrazone.HCl | 0.48 | 1.2 | 1.2 | 10.0* |
| oxycodonhydrazone.HBr | 0.46 | 0.65 | 0.35 | 10.0* |
| oxycodone-thiosemicarbazone ,HCl | 8.5 | 4.5 | - | 100.0 |
| dihydrocodeinone-semicarbazone.HCl | 0.38 | 0.35 | - | 10.0* |
| dihydrocodeinone phenylhydrazone.HCl | 2.4 | 2.3 | - | 10.1** |
| dihydrocodeinone-oxime phosphate | 4.2 | 3.7 | 0.38 | 25.0* |
| dihydrocodeinone thiosemicarbazone.HCl | 1.7 | 0.6 | - | 5.0 |
| morphine | 3.6 | 1.8 | 0.45 | 15.0 |
| oxycodone | 0.92 | 0.68 | 0.45 | 2.2 |
| dihydrocodeinone | 2.2 | 0.98 | 0.9 | 4.9 |

| | | | | |
|---|---|---|---|---|
| *catalepsy | | | | |
| ** convulsion | | | | |

### Methods:

Eur.J.of Pharm.(1982)239-241; Arzn.Forschung 38 (1938) 552. The strongest analgesic in the hot plate test is the dihydrocodeinone-semicarbazone (ED₅₀:0,38 mg/kg hot plate, tail flick). On the algolytic test the oxycodone-oxime, -semicarbazone, -phenylhydrazone and -hydrazone are capable to totally assuage the pain - though amidst the symptoms of slight cathatony - practically in the order of magnitude of morphine or the mother compounds (oxycodone, dihydrocodeinone).

### III.2. Tests on sedative activity

Inactine (35 mg/kg) was used as base narcotic.

**Table III/2**

| Sedative (narcosis potentiating) effect (ED₅₀₀%) s.c. on rats | |
|---|---|
| compound | ED₅₀₀% (mg/kg*) |
| oxycodone-hydrazone.HBr | 0.45 |
| oxycodone oxime phosphate | 0.5 |
| oxycodone-semicarbazone.HCl | 1.5 |
| oxycodone-phenylhydrazone.HCl | 1.5 |
| dihydrocodeinone-phenylhydrazone.HCl | 3.5 |
| dihydrocodeinone-semicarbazone bitartarate | 1.0 |
| morphine | 1.75 |
| dihydrocodeinone | 1.9 |
| oxycodone | 1.2 |

| | |
|---|---|
| * ED₅₀₀%= dose increasing narcosis to five times that of the control. | |
| The doses of the active ingredients are always calculated as base. | |

### Evaluation:

All investigated compounds potentiate the activity of Inactine strongly. The duration of narcosis is most effectively prolongated by oxycodone-hydrazone and -oxime, respectively.

### III.3. Investigation of physical dependency

**Table III/3**

| "jumping test" on mice | | | | |
|---|---|---|---|---|
| compound | treatment dose 7 x i.p. (mg/kg) | naloxone 50 mg/kg i.p. | number of jumps /mouse (average) | percentage of jumping animals (%) |
| morphine | 100 | + | 34.5 | 100 |
| oxycodone-oxime.phosphate | 2 | + | 2.7 | 70 |
| | 5 | + | 2.5 | 50 |
| oxycodone-hydrazone.HBr | 1 | + | 1.5 | 50 |
| The dose of the active ingredient is calculated as base. | | | | |

Table III/3. shows the results of an informative physical dependency study through the jumping test on mice. The mice were treated for three days with a total of 7 times i.p. with a high dose of the compounds to be tested and after the final injection the symptoms of withdrawal are generated by administration of a morphine antagonist, i.e., a constraint for jumping is provoked. Morphine-equivalent quantities were administered for the test compounds. In the case of morphine the dose which effects dependence with certainty amounts to 7x100 mg/kg. Within this group upon administration of 50 mg/kg of naloxone the average jumping rate amounted to 34.5 (total jumping number/jumping animals), and 100 % of the investigated animals did jump. Oxycodone-semicarbazone-oxime and -hydrazone, respectively, when administered in equi-analgetic doses did result only in a very low, almost negligible number of jumping reactions, with 50-70% of the animals participating. The capacity of dependence of these compounds is thus unusually low.

### III.4. Morphine toxicity tests

Morphine and the test substances were administered together, s.c. to rats. As seen from Table III/4. almost twice as much is needed from morphine to develop the same mortality, which means that the toxicity of morphine is decreased to almost its half as a result of the activity of effective test substances (oxycodone-oxime, -semicarbazone, -phenylhydrazone, -hydrazone, and dihydrocodeinone-oxime). Dihydrocodeinone-semicarbazone and -phenylhydrazone are less active. Oxycodone and dihydrocodeinone potentiate the toxicity of morphine (!).

**Table III/4**

| Change of morphine toxicity in the presence of test substances (on rats) | | | |
|---|---|---|---|
| compound | dose (mg/kg, s.c.) | morphine LD₅₀ (mg/kg s.c.) | ratio LD₅₀/ morphine LD₅₀ |
| oxycodone-oxime phosphate | 15 | 620 | 2.2 |
| oxycodone-semicarbazone bitartarate | 50 | 580 | 2.0 |
| oxycodone hydrazone.HBr | 10 | 650 | 2.3 |
| dihydrocodeinone-phenylhydrazone.HCl | 50 | 500 | 1.7 |
| dihydrocodeinone oxime. phosphate | 25 | 750 | 2.6 |
| oxycodone | 10 | 160 | 0.57 |
| dihydrocodeinone | 10 | 150 | 0.53 |
| morphine | - | 280 | 1.0 |
| The doses of the active substances are calculated as base. | | | |

### III.5. Effect in respiration tests on rabbits

### Method:

The investigations were carried out on conscious rabbits of both sexes and of 3-4 kg body weight using a Marey drum. Frequency and amplitude of respiration were quantitatively evaluated.

Morphine decreased both frequency and volume of respiration in doses of 2,5 - 5 mg/kg. Some results are shown in Table III/5.

**Table III/5**

| Effects on respiration on conscious rabbits | | | | |
|---|---|---|---|---|
| compound | dose (µg/kg s.c.) | respiration volume (frequency/min) | | respiration effect |
| morphine.HCl | 2500-5000 i.v., s.c. | ↓↓ | ↓↓ | depression |
| dihydrocodeinone .HCl | 10 - 5500 i.v., s.c. | ↓ | ↓ | depression |
| oxycodone.HCl | 10- 2500 i.v., s.c. | ↓ | ↓ | depression |
| oxycodone-semicarbazone.HCl | 100-500 + morphine* | ↑↓ | ↑ | morphine-inhibition |
| oxycodone-hydrazone.HCl | 50 | | | |
| | 50 (administered after morphine*) | ↑ | ↑ | slight reversion |
| oxycodone-phenylhydrazone.HCl | 100-5000 | 0 | 0 | no change |
| | 1000 | ↑ | ↑ | slight stim. |

| | | | | |
|---|---|---|---|---|
| * Morphine dose in combinations 5 mg/kg. | | | | |

### III.6. Effect in respiration tests on conscious rabbits

### Method:

The method as described above in Example III.5 was used on conscious rabbits of 2,5-3 kg body weight. Registration was continued for 3-4 hours. The samples were injected under the skin of the neck, and the respiration parameters were registered at intervals of 10 minutes.
Type A test: Determination of the dose-effect diagram of the substances ("low dose region" and "high dose region") between 0.01 to 2.5 mg/kg alone, and after selection of particular dose values investigation of morphine prevention.
Type B test: The test was carried out as follows: 5 mg/kg of morphine were administered (causing with certainty respiratory depression), and after 10 -20 minutes it was investigated whether the depression could be reversed.
Type C test: Administration of 5 mg/kg of morphine and the investigated substance together to clarify whether the substance is capable to reduce the effect of morphine.

Some of the results are shown in the following tables.

**Table III/6**

| Oxycodone-oxime (OX) | | | | | |
|---|---|---|---|---|---|
| OX dose (mg/kg s.c.) | pretreatment period (min) | morphine (mg/kg s.c.) | pretreatment period (min) | respiration change | |
| | | | | frequency (min⁻¹) | amplitude |
| 0.005 | 30 | - | - | +30 | +30 |
| 0.1 | 30 | - | - | +47 | +40 |
| 0.025 | 30 | - | - | +28 | 0 |
| 0.5 + naloxone | 10 | - | - | -50 | -50 |
| 0.25 | 10 | - | - | +50 | +50 |
| - | - | 0.5 | 30 | -15 | 0 |
| - | - | 1.0 | 30 | -20 | 0 |
| - | - | 2.5 | 30 | -39 | -20 |
| - | - | 5.0 | 30 | -47 | -80 |
| | | | 120 | -66 | -80 |

| A. | | | | | |
|---|---|---|---|---|---|
| 0.1 | 10 | - | - | +57 | +20 |
| | | + 5 | 30 | +71 | 0 |
| | | | 40 | -29 | -40 |
| pretreatment period = duration of pretreatment | | | | | |

**Table III/7**

| Oxycodone-semicarbazone (OS) | | | | | |
|---|---|---|---|---|---|
| OS dose (mg/kg s.c.) | pretreatment period (min) | morphine (mg/kg s.c.) | pretreatment period (min) | respiration change | |
| | | | | frequency (min⁻¹) | amplitude |
| 0.01 | 10 | - | - | +48 | 0 |
| | 30 | - | - | +51 | +20 |
| 0.025 | 30 | - | - | +51 | +20 |
| 0.05 | 10 | - | - | +50 | +75 |
| 0.025 | 10 | - | - | +48 | +20 |
| | 20 | 5 | 20 | +24 | -16 |
| | 30 | | 30 | +13 | -20 |
| 0.50 | 10 | - | - | +36 | 0 |
| | 30 | 20 | 20 | +10 | 0 |

**Table III/8**

| Oxycodone-phenylhydrazone (OP) | | | | | |
|---|---|---|---|---|---|
| OP dose (mg/kg s.c.) | pretreatment period (min) | morphine (mg/kg s.c.) | pretreatment period (min) | respiration change | |
| | | | | frequency (min⁻¹) | amplitude |
| A. | | | | | |
| 0.1 | 10 | - | - | +31 | +20 |
| 0.5 | 10 | - | - | +16 | 0 |
| | | 5 | 10 | -17 | -20 |
| | 60 | | | 0 | 0 |

| B. | | | | | |
|---|---|---|---|---|---|
| - | - | 5 | 10 | -40 | -40 |
| 1.0 | | | 20 | -16 | +15 |
| | | | 30 | - 6 | +20 |

| C. | | | | | |
|---|---|---|---|---|---|
| 0.25 | | 5 | 20 | +44 | +20 |
| | | | 30 | +92 | +30 |
| 0.25 | | 10 | 10 | +23 | +20 |
| 0.25 | | 20 | 30 | -8 | 0 |

**Table III/9**

| Oxycodone-hydrazone (OH) | | | | | |
|---|---|---|---|---|---|
| OH dose (mg/kg s.c.) | pretreatment period (min) | morphine (mg/kg s.c.) | pretreatment period (min) | respiration change | |
| | | | | frequency (min⁻¹) | amplitude |
| A. | | | | | |
| 0.25 | 20 | - | | +150 | +20 |
| | 30 | - | | +127 | +20 |
| | | 5 | 10 | - 36 | -30 |

| C. | | | | | |
|---|---|---|---|---|---|
| 0.25 | | 5 | 20 | + 30 | +10 |

**Table III/10**

| Dihydrocodeinone-phenylhydrazone (DP) | | | | | |
|---|---|---|---|---|---|
| DP dose (mg/kg s.c.) | pretreatment period (min) | morphine (mg/kg s.c.) | pretreatment period (min) | respiration change | |
| | | | | frequency | amplitude |
| A. | | | | | |
| 0.01 | 10 | - | - | + 85 | 0 |
| | 20 | - | - | + 96 | 0 |
| 0.025 | 10 | - | - | +114 | 0 |
| | 20 | - | - | +114 | 0 |
| 0.05 | 10 | - | - | +132 | 0 |

| C. | | | | | |
|---|---|---|---|---|---|
| 0.25 together | | 5 | 10' | + 39 | 0 |
| | | | 20' | + 19 | 0 |
| | | | 30' | - 3 | 0 |
| | | | 50' | - 4 | 0 |

The Table shows that doses of 0.01 to 0.05 mg/kg considerably increase the frequency without influence on the amplitude of respiration. Doses of 0.25 mg/kg when administered together with morphine retard depression to some degree.

### IV. Chemical synthesis examples

### (Method: HU-A-199 901)

### IV.1. Dihydrocodeinon-oxime

1.5 g of hydroxylamine-hydrochloride are allowed to react in 70 ml of water with 3.0 g of dihydrocodeinone base for 3 h; thereafter, the pH is adjusted to 9-10. The oxime precipitates as crystals, which are separated by filtration, washed with water and crystallized from propanol. The product yield is 2.5 g. M.p.: 265 - 266°C.

### IV.2. 14-Hydroxydihydrocodeinone-oxime

The product of Example IV.1. is crystallized from aqueous ethanol. M.p.: 194°C.

### IV.3. 14-Hydroxydihydrocodeinone-hydrazone (trans)

5 ml of 100 % hydrazine hydrate are heated with 2.0 g of 14-hydroxydihydrocodeinone in 10 ml of dimethylformamide for 2 h and then poured into water. The crystalline product which precipitates is isolated and subjected to chromathography on silica gel (chloroform-methanol 9:1,v/v). The pure fractions obtained by TLC eluating with a chloroform: methanol: conc. ammonium hydroxide mixture 90:10:5 as eluent are crystallized from methanol. M.p.:192- 194°C.

### IV.4. 14-Hydroxycodeinone-hydrazone

Using the method of Example IV.3., 1.8g 14-hydroxycodeinone-hydrazone are obtained from 2.0g of 14-hydroxycodeinone. The crude product contains two components as shown by TLC using the above chloroform: methanol: conc. ammonium hydroxide mixture 90:10:5 as eluent. The main component can be obtained in pure form by way of preparative thin-layer chromatography. Crystallized from ethanol:m.p.: 212- 215°C.

### IV.5. 14-Hydroxydihydrocodeinone-semicarbazone (trans)

According to the method described in Example IV.4. 3.2g of the crude product are obtained from 3.0 g of 14-hydroxydihydrocodeinone, which is a mixture of the syn and anti isomers (cis:trans = 1:1). Through crystallization (chloroform - ethanol) the pure trans semicarbazone is obtained. M.p.: 236- 238°C.

### IV.6. 14-Hydroxydihydrocodeinone-phenylhydrazone (cis)

Starting from 1.55g of 14-hydroxydihydrocodeinone 1.7g of 14-hydroxydihydrocodeinone-phenylhydrazone are obtained with the process given in Example IV.3. M.p. after recrystallization: 174 - 176°C. Pure cis isomer.

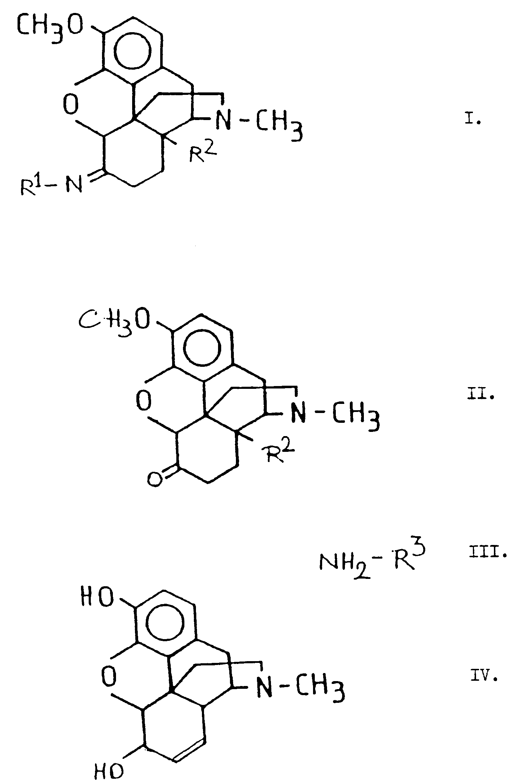

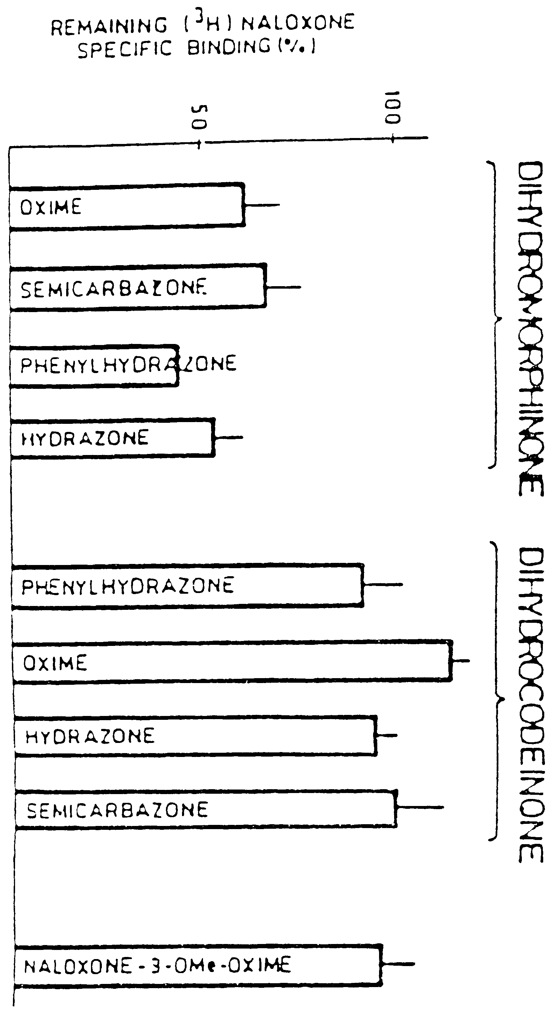

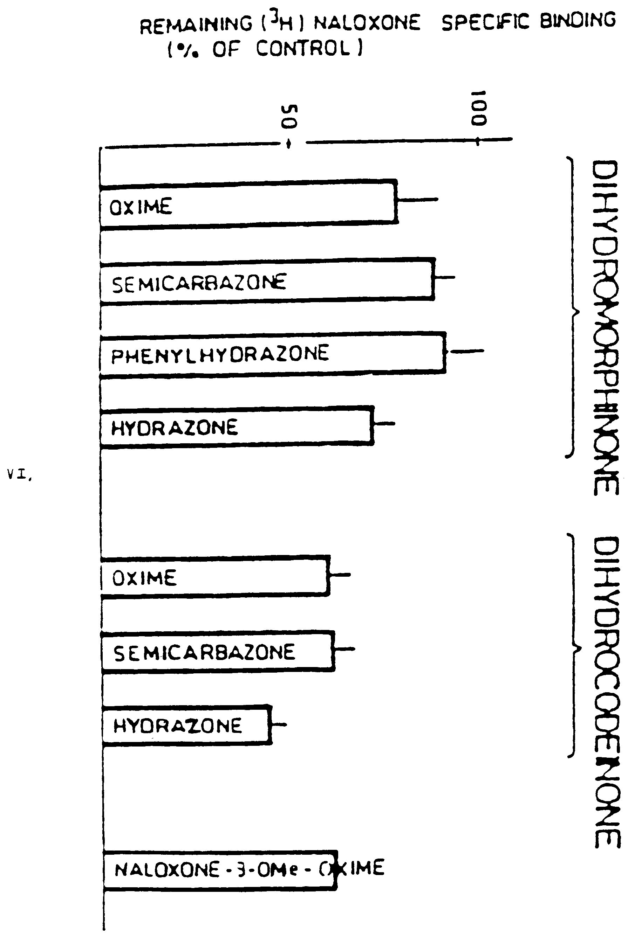

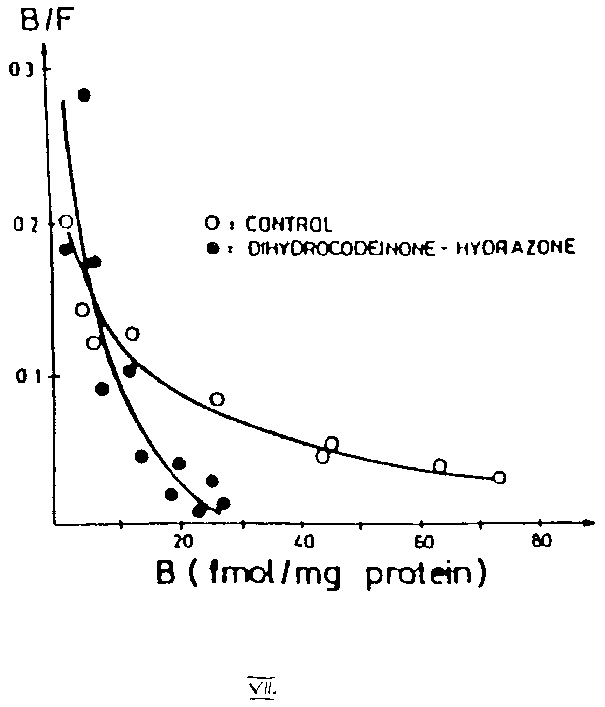

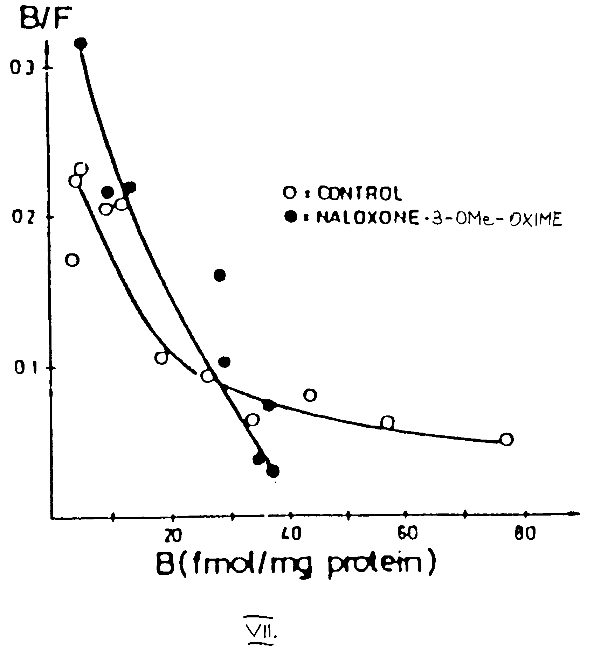

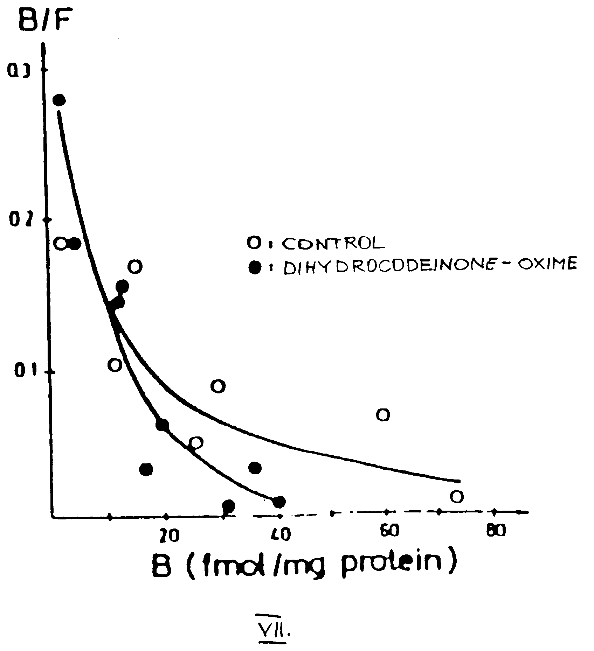

## Claims

1. Codeinone derivatives or the general formula Ia, 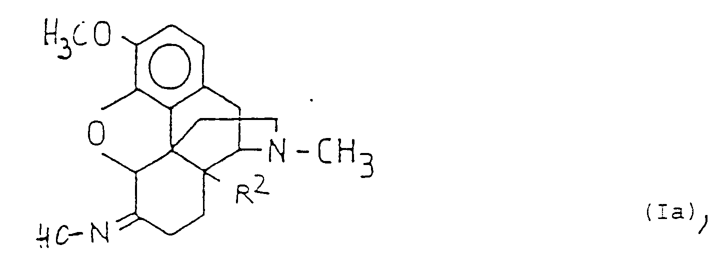 wherein
R² is a hydrogen atom or hydroxy,
and their salts.

2. Pharmaceutical compositions comprising a morphinane derivative,
characterized in that
the morphinane derivative is a codeinone derivative of formula Ia according to claim 1.

3. Codeinone derivatives of formula Ia according to claim 1 and pharmaceutical compositions according to claim 2,
characterized in that
the codeinone derivatives of formula Ia are present in the form of their cis and/or trans isomers.

4. Pharmaceutical compositions according to claim 2 and/or 3
exhibiting analgetic activity,
characterized in that they comprise
(A) a codeinone derivative of the general formula Ia or a salt thereof,
and
(B) morphine of the formula IV 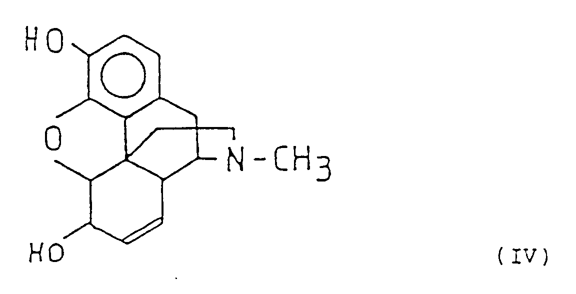 or
another opiate of agonist type or an opioid compound,
the codeinone derivative of component (A) being present in an amount of 2 to 3 parts by mass per 1 part by mass of morphine or a biologically equipotent amount of non-morphine component (B).

5. Process for the preparation of the codeinone derivatives of the general formula Ia according to claim 1,
characterized in that
in a manner known as such, a ketone of the general formula II, 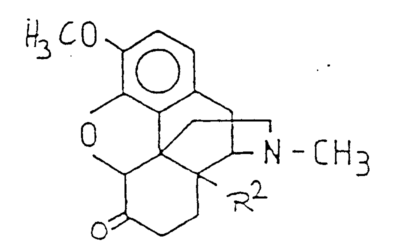 wherein
R² is a hydrogen atom or hydroxy,
is allowed to react with a hydrazine derivative of the general formula IIIa,
H₂N-R^{3'} (IIIa),
wherein
R^{3'} is hydroxy or
a group capable of being transformed into hydroxy.

6. Use of codeinone derivatives of the general formula I, 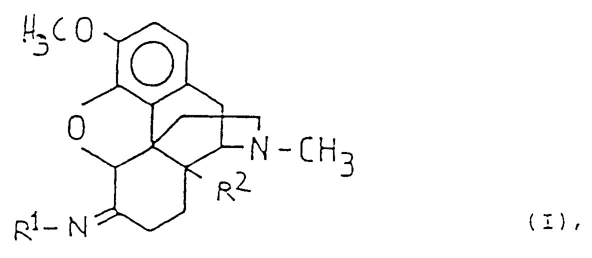 wherein
R¹ is amino, hydroxy, -NH-phenyl or -NH-CO-NH₂,
and
R² is a hydrogen atom or hydroxy,
including their salts,
for preparing pharmaceutical compositions for analgetic treatment without respiratory depression.

## Patentansprüche

1. Codeinonderivate der allgemeinen Formel Ia, 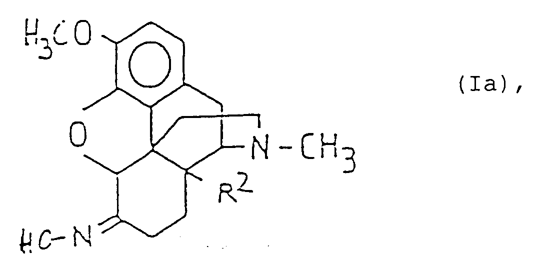 worin R² ein Wasserstoffatom oder Hydroxy bedeutet,
sowie ihre Salze.

2. Pharmazeutische Zusammensetzungen, die ein Morphinanderivat enthalten, dadurch gekennzeichnet, daß das Morphinanderivat ein Codeinonderivat der Formel Ia nach Anspruch 1 ist.

3. Codeinonderivate der Formel Ia nach Anspruch 1 sowie pharmazeutische Zusammensetzungen nach Anspruch 2, dadurch gekennzeichnet, daß die Codeinonderivate der Formel Ia in Form ihrer cis- und/oder trans-Isomeren vorliegen.

4. Pharmazeutische Zusammensetzungen nach Anspruch 2 und/oder 3, die analgetische Wirksamkeit aufweisen,
dadurch gekennzeichnet, daß sie enthalten:
(A) ein Codeinonderivat der allgemeinen Formel Ia oder ein Salz davon
und
(B) Morphin der Formel IV 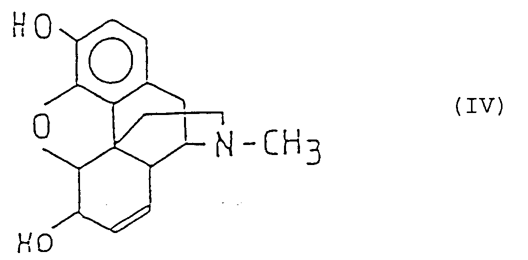 oder
ein anderes Opiat vom Agonistentyp oder eine Opioidverbindung,
wobei das Codeinonderivat der Komponente (A) in einer Menge von 2 bis 3 Masseteilen, bezogen auf 1 Masseteil Morphin oder eine gleicher biologischer Wirksamkeit entsprechende Menge einer Nichtmorphin-Komponente (B), vorliegt.

5. Verfahren zur Herstellung der Codeinonderivate der allgemeinen Formel Ia nach Anspruch 1, dadurch gekennzeichnet, daß in an sich bekannter Weise ein Keton der allgemeinen Formel II, 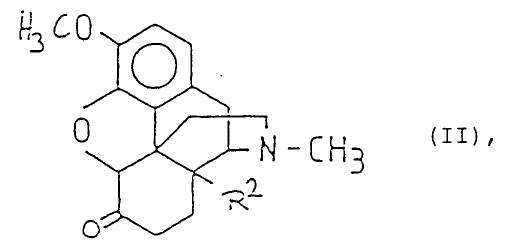 in der R² ein Wasserstoffatom oder Hydroxy bedeutet,
mit einem Hydrazinderivat der allgemeinen Formel IIIa,
H₂N-R^{3'} (IIIa),
in der R^{3'} Hydroxy oder eine Gruppe darstellt, die in Hydroxy umgewandelt werden kann,
umgesetzt wird.

6. Verwendung von Codeinonderivaten der allgemeinen Formel I, 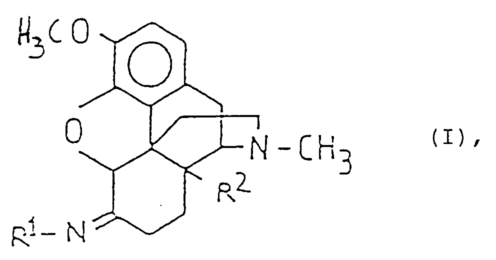 in der bedeuten:
R¹ Amino, Hydroxy, -NH-Phenyl oder -NH-CO-NH₂
und
R² ein Wasserstoffatom oder Hydroxy,
einschließlich ihrer Salze,
zur Herstellung pharmazeutischer Zusammensetzungen zur analgetischen Behandlung ohne respiratorische Depression.

## Revendications

1. Dérivés de cotéinone de formule générale Ia, 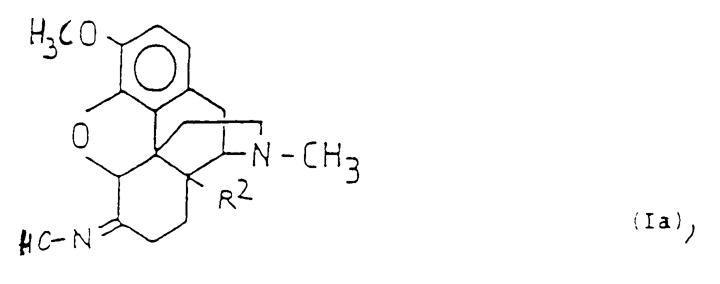 dans laquelle
R² est un atome d'hydrogène ou un groupement hydroxy,
et leurs sels.

2. Compositions pharmaceutiques comprenant un dérivé de morphinane, caractérisées en ce que le dérivé de morphinane est un dérivé de codéinone de formule Ia selon la revendication 1.

3. Dérivés de codéinone de formule Ia selon la revendication 1 et compositions pharmaceutiques selon la revendication 2, caractérisés en ce que les dérivés de codéinone de formule Ia sont présents sous la forme de leurs isomères cis et/ou trans.

4. Compositions pharmaceutiques selon la revendication 2 et/ou 3 présentant une activité analgésique, caractérisées en ce qu'elles comprennent
(A) un dérivé de codéinone de formule générale Ia ou un sel de celui-ci,
et
(B) la morphine de formule (IV) 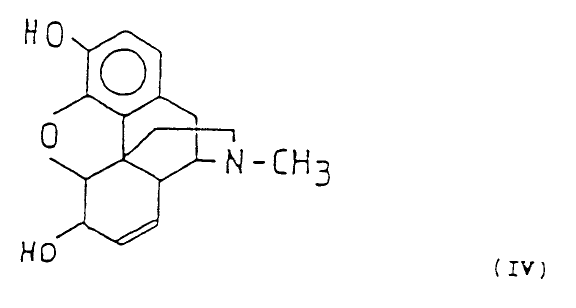 ou
un autre opiacé du type agoniste ou un composé opioïde,
le dérivé de codéinone du composant (A) étant présent dans une quantité de 2 à 3 parties en masse pour une partie en masse de morphine ou d'une quantité biologiquement équipotente de composant non morphine (B).

5. Procédé de préparation de dérivés de codéinone de formule générale Ia selon la revendication 1,
caractérisé en ce que
d'une manière connue en soi, on fait réagir une cétone de formule générale II, 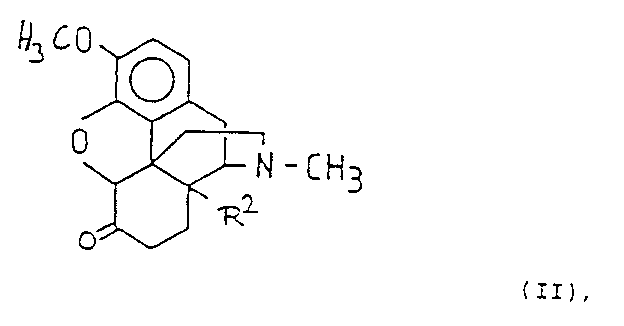 dans laquelle
R² est un atome d'hydrogène ou un groupement hydroxy,
avec un dérivé d'hydrazine de formule générale IIIa,
H₂N-R^{3'} (IIIa),
dans laquelle
R^{3'} est un groupement hydroxy ou
un groupement susceptible d'être transformé en groupement hydroxy.

6. Utilisation de dérivés de codéinone de formule générale I, 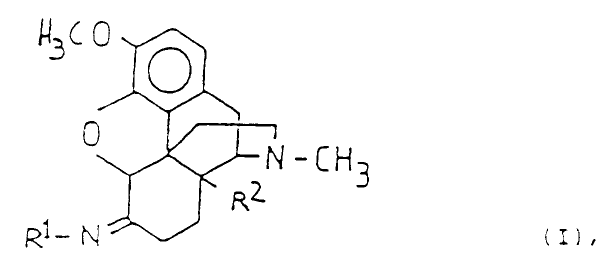 dans laquelle
R¹ est un groupement amino, hydroxy, -NH-phényl ou -NH-CO-NH₂,
et
R² est un atome d'hydrogène ou un groupement hydroxy,
y compris leurs sels,
pour la préparation de compositions pharmaceutiques pour un traitement analgésique sans dépression respiratoire.
